# EUROPEAN PATENT SPECIFICATION

(11) **EP 2 881 034 B1**
(45) Date of publication and mention of the grant of the patent: **10.06.2020**
(21) Application number: 14199419.4
(22) Date of filing: 30.06.2010
(51) Int. Cl.: A61B 5/0205, A61B 5/02, A61B 5/00

(54) **Monitoring and displaying a patient's status**
Überwachung und Anzeige eines Patientenstatus
Surveillance et affichage de l'état d'un patient

(30) Priority: 30.06.2009 US 222101 P; 24.12.2009 US 647198
(43) Date of publication of application: 10.06.2015
(62) Divisional of application: 10794719.4
(73) Proprietor: Edwards Lifesciences Corporation, Irvine, CA 92614 (US)
(72) Inventor: Mckeown, Morgan, Irvine, CA 92614 (US); Michard, Frederic, Irvine, CA 92614 (US); De Jonghe, Ives, Irvine, CA 92614 (US); Roteliuk, Luchy, Irvine, CA 92614 (US); Frazier, John, Irvine, CA 92614 (US); Glines, Erin, Irvince, CA 92614 (US); Doorish, Shane, Irvince, CA 92614 (US); Patton, Doug, Irvince, CA 92614 (US)
(74) Representative: Eisenführ Speiser

(56) References cited:
- US-A1- 2007 050 715
- US-A1- 2009 012 416
- US-A1- 2009 024 008

## Description

### Cross-Reference to Related Application

This application claims the benefit of U.S. Provisional Patent Application Serial No. 61/222,101, entitled "SYSTEMS AND METHODS FOR MONITORING A PATIENT STATUS IN RESPONSE TO A CLINICAL EVENT," filed June 30, 2009.

### BACKGROUND

### Field

The disclosure relates to monitoring vital signs of a patient, and more particularly to systems and methods for monitoring and displaying a patient's status.

### Related Art

Devices for measuring various physiological parameters, or "vital signs," of a patient, such as temperature, blood pressure, heart rate, heart activity, etc., have been a standard part of medical care for many years. Indeed, the vital signs of some patients (e.g., those undergoing relatively moderate to high levels of care) typically are measured on a substantially continuous basis to enable physicians, nurses and other health care providers to detect sudden changes in a patient's condition and evaluate a patient's condition over an extended period of time.

Medical patient monitors are typically employed to provide a variety of physiological patient data to physicians or other health care providers. Such physiological patient data facilitates diagnosis of abnormalities (as monitored in emergency rooms), or the patient's current condition (as monitored in operating rooms or in intensive care units), or permit long-term trend monitoring (such as Holter monitoring or stress testing as part of an annual physical examination).

Presently, one or more sensors (also referred to as transducers) are connected to the patient to acquire various physiological information associated with that patient (e.g., electrical impulses, resistance measurements, etc.). Such physiological information is then processed into physiological data suitable for outputting to the physician or other health care provider. The physiological data can be displayed on a screen or provided on paper in either graphical and/or numerical format. Analog or digital strip chart recorders, spreadsheets and plotting programs are examples of output devices of physiological data. Additionally, the physiological data may be stored in a memory device or transmitted over a network for remote access and/or further processing.

Unfortunately, in order to present a large quantity of physiological data in a single screen in a meaningful manner, data presentation may be presented in less than intuitive fashion (e.g., replacing amplitude geometry with color indexing) and for some aspect of the data deemed to be "unimportant," such data may be omitted or otherwise modified. Some users of the equipment find such display representation to be visually unappealing and may result in slowing down or degrading the clinical usefulness of the acquired data. Moreover, once display of the data has been initiated, users usually have limited ability to interface or manipulate the displayed data to further facilitate the clinical usefulness of the data for that particular user.

In addition, current systems allow only limited recording and displaying of patient parameters. For example, in response to a clinical event such as the administration of a drug, the clinician must constantly monitor the patient display in order to determine a change in patient's status, and must manually make calculations for an exact deviation or change in a patient parameter. The medical patient monitors themselves do not provide an indication of if and to what extent a patient's status may have changed due to the clinical event. Further, the medical patient monitors do not display the patient parameters such that the patient's status can easily be determined.

Therefore, a need exists for an intuitive patient monitoring interface that allows clinicians to more accurately and easily monitor and determine a patient's status.

US 2007/050715 A1 shows data by animated avatars, computer interfaces displaying physiological data by animated avatars.

US 2009/024008 A1 shows an apparatus and a method for monitoring physiological data and displaying the same on a screen by animated graphic elements.

US 2009/012416 A1 shows systems and methods for monitoring lung fluid status.

### SUMMARY

The disclosure relates to an interactive system for more accurately and easily displaying and monitoring a patient's status.

The invention provides a system for providing a physiological representation of a patient according to independent claim 1. The invention provides also a computer readable medium storing a program for providing a physiological representation of a patient according to independent claim 4. The invention is set out in the appended set of claims. The embodiments and/or examples of the following description which are not covered by the appended claims are considered as not being part of the present invention.

### BRIEF DESCRIPTION OF THE DRAWINGS

These and other embodiments of the disclosure will be discussed with reference to the following exemplary and non-limiting illustrations, in which like elements are numbered similarly, and where:
FIG. 1 is a block diagram of the patient monitoring system according to an embodiment of the disclosure;
FIG. 2 is a view of an intervention analysis screen according to an embodiment of the disclosure;
FIG. 3 is a view of a patient parameter screen with indicator displays having an upside-down lantern icon according to an embodiment of the disclosure;
FIG. 4 is a view of a patient parameter screen with cockpit-type indicator displays according to an embodiment of the disclosure;
FIG. 5 is a view of a parameter configuration screen according to an embodiment of the disclosure;
FIG. 6 is a view of a parameter configuration screen according to an embodiment of the disclosure;
FIG. 7 is a view of a screen displaying multiple patient parameters according to an embodiment of the disclosure;
FIG. 8 is a view of an alarm/target configuration screen according to an embodiment of the disclosure;
FIG. 9 is a view of an alarm/target configuration screen according to an embodiment of the disclosure;
FIG. 10 is a view of a physiological indicator display according to an embodiment of the disclosure;
FIG. 11 is a flow diagram of the event marking and analysis method according to an embodiment of the disclosure;
FIG. 12 is a view of a physiological indicator indicating an increased heart size according to an embodiment of the disclosure;
FIG. 13 is a view of a physiological indicator indicating a decreased heart size according to an embodiment of the disclosure;
FIG. 14 is a view of a physiological indicator indicating a lung with fluid according to an embodiment of the disclosure;
FIG. 15 is a view of a physiological indicator indicating a lung with fluid according to an embodiment of the disclosure;
FIG. 16 is a view of a physiological indicator indicating a lung with fluid according to an embodiment of the disclosure;
FIG. 17 is a view of a physiological indicator indicating blood circulation based on cardiac output according to an embodiment of the disclosure;
FIG. 18 is a view of a physiological indicator indicating blood circulation based on cardiac output according to an embodiment of the disclosure;
FIG. 19 is a view of a physiological indicator indicating blood circulation based on cardiac output according to an embodiment of the disclosure;
FIG. 20 is a view of a physiological indicator indicating vascular track shrinkage according to an embodiment of the disclosure;
FIG. 21 is a view of a physiological indicator indicating vascular track growth according to an embodiment of the disclosure;
FIG. 22 is a view of a physiological indicator including a stroke volume variation starling curve according to an embodiment of the disclosure;
FIG. 23 is a view of a physiological indicator including a stroke volume variation starling curve according to an embodiment of the disclosure;
FIG. 24 is a view of a physiological indicator including a stroke volume variation starling curve according to an embodiment of the disclosure; and
FIG. 25 is a view of a physiological indicator including a physiological relationship screen according to an embodiment of the disclosure.

### DETAILED DESCRIPTION

Apparatus, systems and methods that implement the embodiments of the various features of the disclosure will now be described with reference to the drawings. The drawings and the associated descriptions are provided to illustrate some embodiments of the disclosure and not to limit the scope of the disclosure. Throughout the drawings, reference numbers are re-used to indicate correspondence between referenced elements.

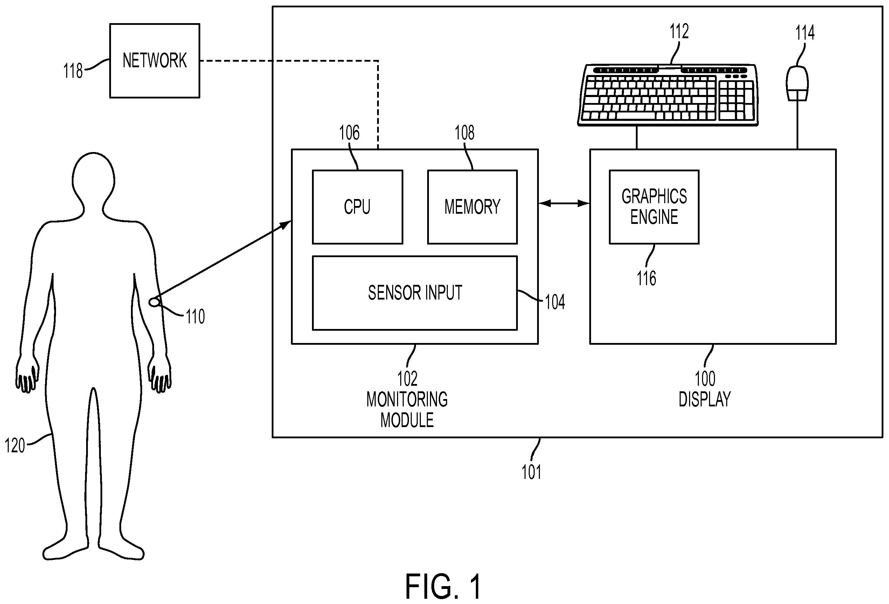

FIG. 1 is a diagram of a patient monitoring system 101 according to an embodiment of the disclosure. The patient monitoring system 101 includes at least one sensor 110 attached to a patient 120. In a preferred embodiment, the patient monitoring system 101 is a bed-side system, and can be integrated into an existing drug delivery stand, bedbox, or monitoring system rack. The sensor 110 is coupled to a monitoring module 102. The monitoring module 102 includes a central processing unit (CPU) 106, a memory 108, and sensor input circuitry 104. In an embodiment, the monitoring module 102 is connected to a network 118, such as a wired or wireless network, to allow monitoring on a remote display (not shown). The memory 108 can be a volatile memory, such as flash memory, or non-volatile memory, such as read-only memory. In addition, the memory 108 can be a database that is located within the system 101, or alternatively, located remotely from the system 101. In another embodiment, the memory can be located within or coupled to a display 100.

The monitoring module 102 is coupled to the display 100. The monitoring module 102 receives raw physiological data from the patient 120, and converts the raw data into graphical or textual signals, and then transmits these signals to the display 100. The display 100 includes a graphics engine 116 which renders the signals received from the monitoring module 102, and outputs images and graphics corresponding to the raw physiological data to the display 100. In an embodiment, the display 100 is touch-sensitive, and allows data or commands to be entered by an application of pressure, via, for example, a clinician's finger or a stylus, to the display 100. Furthermore, the display 100 can include a keyboard 112 for data input. The keyboard 112 can be a touch sensitive keyboard located on a portion of the display 100, or it can be an external hard keyboard coupled to the display 100. A mouse or pointing device 114 can be coupled to the display 100 and used to enter data or commands into the system 101.

In an embodiment, the display 100 and the monitoring module 102 can be an integrated unit with a single housing. In another embodiment, the monitoring module 102 can be separate from the display 100.

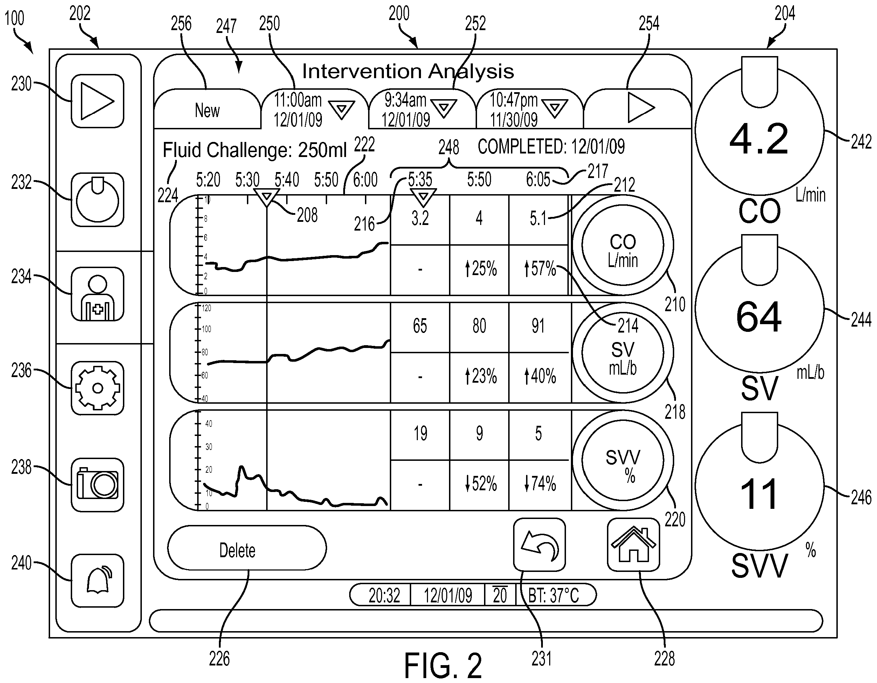

FIG. 2 is a view of an intervention analysis screen 200 for event marking and displaying percentage change information. The intervention analysis screen 200 is shown on the display 100. The screen 200 includes a left panel 202 that includes navigation buttons 230-240 and a right panel 204. In an embodiment, the navigation buttons 230-240 include a trigger button 230, a parameter configuration button 232, a patient monitor button 234, a settings button 236, a screen capture button 238, and an alarm button 240. Each button navigates the clinician to a respective screen. For example, upon selection of the patient monitor button 234, the intervention analysis screen 200 is displayed between the left panel 202 and the right panel 204.

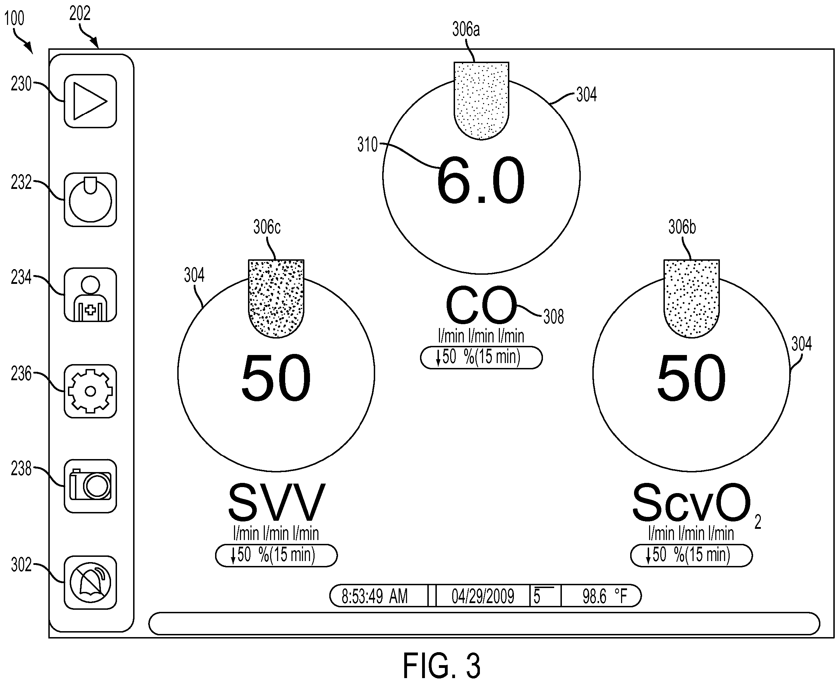

In an embodiment, the right panel 204 displays real-time patient vital signs on the indicator displays 242 - 246. For example, the cardiac output indicator display 242 displays the patient's current cardiac output reading. The right panel 204 can include any number of indicators, and the number of indicators displayed can be configured by the clinician through a parameter configuration screen, which is displayed when the clinician selects the parameter configuration button 232. The indicator displays are described in more detail in FIG. 3.

The intervention analysis screen 200 allows the clinician to view multiple parameters, such as cardiac output (CO), stroke volume (SV), and stroke volume variation (SVV) on a single display. For each parameter, a time-lapse graph 222 is provided, as well as a table 248 showing a change in the parameter value over time.

The clinician can set a reference point 208 by inputting the start time and type of intervention. The reference point 208 can indicate the occurrence or start of a clinical event, such as, but not limited to, the administration of a drug, a fluid challenge, a change in patient care, physically moving or adjusting the patient's position, and/or passive arm or leg raises. The intervention selected can depend on a patient's situation and the types of intervention which are critical to the care of the patient. The reference point 208 provides advantages to the clinician over conventional systems by allowing the clinician to view when the intervention begins and also all effects of the intervention after the intervention began. Thus, the clinician does not need to memorize when the intervention began, or any base measurements for the intervention. Furthermore, the clinician does not need to perform any calculations to ascertain the benefits provided by the intervention. In an embodiment, the clinician can manually enter the type of clinical event, using a soft keyboard integrated with the display 100, or via an externally coupled hard keyboard. In one embodiment, a title 224 (e.g., fluid challenge) of the clinical event is displayed on the screen 200. In another embodiment, an icon (e.g., fluid challenge) is displayed representing the clinical event.

Once the reference point 208 is set, the system 101 monitors changes in each parameter value and displays the changes in the table 248. Advantageously, this feature allows the clinician to quickly and easily determine a patient's status. Table 248 summarizes the effect of the clinical event on various patient vital signs. For example, as shown in FIG. 2, the reference point 208 is set at 5:35, which represents a point in time. Referring to the CO parameter display 210, the initial value 216 for CO when the reference point 208 was set is 3.2 L/min. At 6:05, thirty minutes later, the later value 212 for CO is 5.1 L/min., representing a 57% increase in the patient's CO value. The percentage change indicator 214 displays this 57% percent increase of the patient's CO value from 5:35 to 6:05. The percentage change indicator 214 includes an arrow indicating if the percentage change is negative or positive. In an embodiment, the change indicator 214 can be in specific measurable units instead of a percentage value.

In an embodiment, the percentage change can be calculated and displayed in table 248 every fifteen minutes as shown in FIG. 2. The percentage change can also be calculated and displayed in table 248 according to a clinician-selected frequency, such as every second, every ten seconds, every minute, every hour, every day, every week, etc. In another embodiment, the percentage change can be calculated and displayed upon the occurrence of a clinical event, such as, for example, a drip from a drug delivery drip bag, or the patient having a meal. In another embodiment, the absolute value of a parameter or change in parameter value, or the absolute percentage change within a previous time segment is calculated and displayed.

In another embodiment, the system 101 allows the clinician to follow the progress of a patient by variables such as current and historical parameter values, continuous percentage change over a rolling selectable time period, and a discrete percentage change over a clinical event period.

In one embodiment, the percentage change indicator 214 and value 212, at a subsequent time period, can be displayed in a first color, such as a green color, if the values increase from the initial value 216. However, the percentage change indicator 214 and the value 212, at a subsequent time period, can be displayed in a second color, such as a yellow color, if the value remains relatively stagnant from the initial value 216. Furthermore, the percentage change indicator 214 and the value 212, at a subsequent time period, can be displayed in a third color, such as a red color, if the value decreases from the initial value 216. The first color, the second color, and/or the third color may be selected such that they are sufficiently different from each other and have high degrees of contrast to each other. In one embodiment, the first color can be selected such that it is associated with a calm or OK feeling, while the second color can be selected such that it is associated with a cautious feeling, and the third color can be selected such that it is associated with a danger feeling.

In an embodiment, the reference point 208 is set for all of the parameters, such as CO, SV, and SW as shown in FIG. 2. Thus, for each parameter, the percentage changes indicated in each parameter's respective table is based on the common reference point 208. In another embodiment, a separate reference point 208 can be set for each parameter. For example, the reference point for SV can be set at 5:20, while the reference point for SVV can be set at 5:40. This feature is useful if multiple clinical events occur at different times, and each clinical event has an affect on a different parameter.

In an embodiment, the screen 200 includes tabs 247 which allow the clinician to view patient data from different time periods. For example, tab 250 displays the current patient data as of 11:00 a.m. Selecting tab 252 displays patient data from 9:34 a.m. Selecting tab 254 scrolls the screen 200 to the right and displays additional tabs for different time periods. Selecting the "New" tab 256 allows the clinician to record a new patient monitoring session.

In an embodiment, the screen 200 also includes a home button 228 which navigates the clinician to a "Home" screen. The "Home" screen can include patient information, a summary of a patient's vital signs, and/or a graph monitoring patient parameters in real-time. The screen 200 can also include a back button 231, which navigates the clinician to the previously viewed tab containing patient data.

After a patient monitoring session is complete (e.g., patient data is completely acquired for a desired time period), the data is automatically saved to the memory 108. If the clinician does not wish to save the patient monitoring session, then the delete button 226 can be selected, which removes the data from the session from the memory 108. In addition, if the clinician navigates to a previously stored patient data tab, such as tab 250, selecting the delete button 226 removes the patient data corresponding to tab 250 from the memory 108.

FIG. 3 is a view of a patient parameter screen with indicator displays having an upside-down lantern shaped icon 306. Although in FIG. 3, the icon 306 is an upside-down lantern, the icon 306 can also be of any shape in any orientation which is large, easily visible, and provides contrast with the adjacent circle. Indicator displays, advantageously, allow the clinician to quickly and easily determine a patient's status. The indicator displays is easily identifiable and allows the clinician to view a status of the patient without having to read the numbers and correlate the numbers to a specific range of acceptable values. In an embodiment, the indicator displays include the upside-down lantern shaped icon 306, a patient value reading 310, and the name of the monitored parameter 308. In an embodiment, the lantern shaped icon 306 has three colors. When the patient value reading 310 is within a "normal" range, as defined by the clinician, or pre-determined and stored in the memory 108, the icon 306 has a first color. If the patient value reading 310 is nearing an alarm threshold, the icon 306 changes to a second color. Finally, if the patient value reading 310 reaches or surpasses the alarm threshold, then the icon 306 changes to a third color. The first color, the second color, and the third color, may be, for example, green, yellow, and red, respectively, or any other color.

The indicator configuration screen can include any number of indicator displays, and is not limited to displaying three indicator displays 304 as show in FIG. 3. In one embodiment, the monitored parameters 308 include stroke volume variation (SW), cardiac output (CO), central venous saturation (ScvO₂), and systemic vascular resistance index (SVRI). In hemodynamic monitoring, it may be critical to analyze oxygen output and consumption by the organs. Thus, the CO and the ScvO₂ value may be important to hemodynamic monitoring since the CO corresponds to oxygen output by the organs while the ScvO₂ corresponds to oxygen consumption by the organs. Furthermore, the SW may be important since it can indicate whether fluid treatment can increase cardiac output or not. The SVRI may allow normalization of the vascular resistance for people with different heights and/or weights.

In another embodiment, the icon 306 can display a different color and/or a different shade of the same color for each of the statuses: normal, nearing an alarm threshold, and reaching the alarm threshold. The different shading can allow for situations where the status of the patient isn't binary such as good or bad, but instead has gray areas where the status of the patient is between good and bad. This allows the clinician to make a determination of the patient's status based upon the clinician's preference or the hospital's preference. In another embodiment, the icon 306 can blink at a first pace when the patient value reading 310 is nearing an alarm threshold, and can blink at a faster second pace when the patient value reading 310 reaches or surpasses an alarm threshold. Furthermore, if the patient value reading 310 reaches or surpasses the alarm threshold, the system 101 may emit audible tones or warnings. The alarm can also be turned off 302 by toggling the alarm button 240 in the left panel 202 of the display 100.

The clinician can access the indicator configuration screen by selecting the parameter configuration button 232 in the left panel. The indicator configuration screen further provides the clinician with an intuitive graphical clinician interface that allows the clinician to easily select which parameters will be displayed, how the parameters will be displayed, such as, for example, color, tone, shading, contrast, brightness, size, shape, etc. The interface with pictures allows the clinician to easily identify parameters to be displayed since humans may more readily identify images instead of text or numbers. Furthermore, the color, tone, shading, contrast, brightness, size, and/or shape can be customized to the clinician's preferences to allow the clinician to determine how the images are displayed so as to improve the clinician's recognition of the parameters.

In another embodiment, indicator displays 304 also illustrate additional information besides the patient value reading 310. For example, similar to table 248, the indicator displays 304 can also include a percentage change between a reference point and the patient value reading 310, the time elapsed since the reference point, and an arrow indicating if the percentage change is negative or positive.

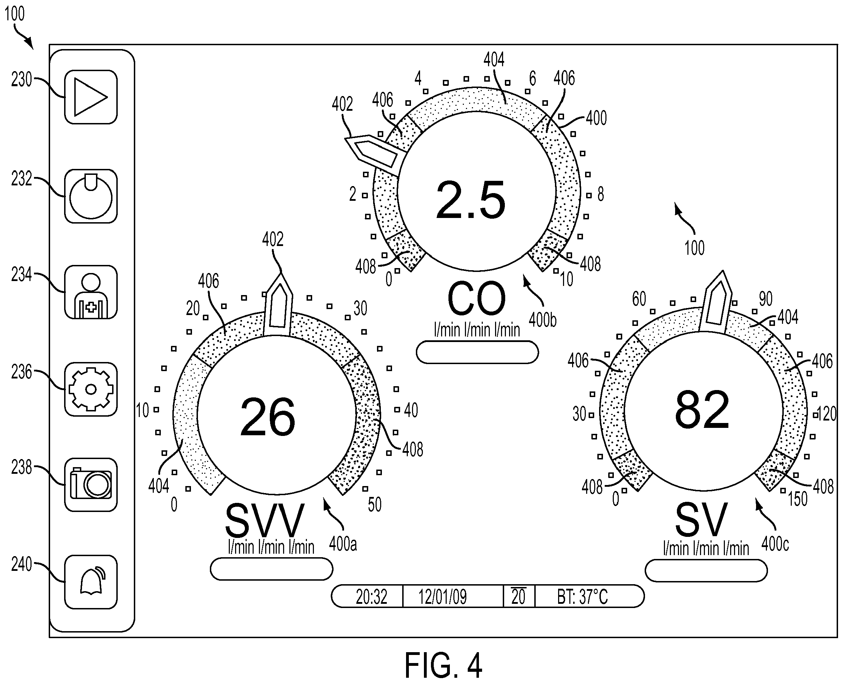

FIG. 4 is a view of a patient parameter screen with cockpit-type indicator displays 400. Each cockpit-type indicator display 400 includes an indicator needle 402. Each display 400 includes multiple status regions. In an embodiment, each cockpit-type indicator display 400 has three colors. For example, the SW indicator display 400a includes a first area 404, a second area 406, and a third area 408. The first area 404 can be, for example, a "normal area" in the first color, such as green. The second area 406 can be, for example, an "alert" area in the second color. The third area 408 can be, for example, an "alarm" area in the third color. The first color, the second color, and the third color, can be, for example, green, yellow, and red, respectively. As the patient parameter value increases or decreases, the indicator needle 402 moves in a corresponding direction around the indicator displays 400. For example, in the SVV indicator display 400a, the indicator display 402 moves counter-clockwise as the status of the SVV deteriorates and clockwise as the status of the SW improves. This may be beneficial in situations where one extreme value is indicative of a healthy patient and the opposite extreme value is indicative of an unhealthy patient. However, in the CO indicator display 400b and the SV indicator display 400c, the indicator display 402 stays in the first area 404 for the normal area and moves to the second area 406 or the third area 408 as the conditions deteriorate. This may be beneficial in situations where values within a first area is indicative of a healthy patient, and values which are below or above the first area are indicative of an unhealthy patient. In one embodiment, in addition to the colors illustrated in the status regions, a color corresponding patient value reading is illustrated around the patient value reading.

For the SW indicator display 400a, the low values for SW are normal and the high values are not. For other indicators, such as the CO indicator display 400b or the SV indicator display 400c, an abnormally high or low value would be abnormal. For the CO indicator display 400b or the SV indicator display 400c, a normal area can be centered around a particular value with an alert area surrounding the normal area, and an alarm area surrounding the alert area.

When the indicator needle 402 is in the clinician-defined normal area 404, the patient parameter value is within a target range. When the indicator needle 402 is in the alert area 406, the patient parameter value is in an alert range, indicating to the clinician that action may be necessary. Finally, when the indicator needle 402 is in the alarm area 408, the patient parameter value is in an alarm range, indicating to the clinician that action may be urgently required. The colors at the junction of each status area may be clearly defined, or may bleed together to give a blended color perception. The colors may bleed together or give a blended color perception can allow for situations where the status of the patient is not binary such as good or bad, but instead has gray areas where the status of the patient is between good and bad. This allows the clinician to make a determination of the patient's status based upon the clinician's preference or the hospital's preference.

In an embodiment, when the indicator needle 402 is in the alarm area 408, the system 101 may emit audible tones or warnings. Furthermore, the display 400 or the indicator needle 402 may blink when the indicator needle is in the alert area 406 or the alarm area 408.

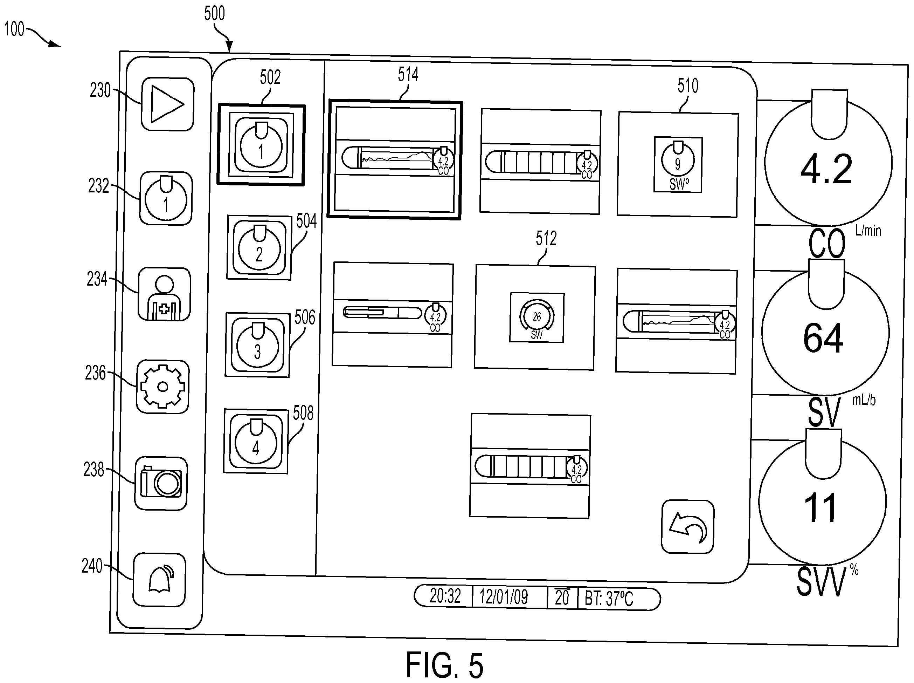

FIGS. 5-6 illustrate a methodology for scaling and configuring parameters displays. FIG. 5 is a view of a parameter configuration screen 500 corresponding to a single parameter being selected to display. The parameter configuration screen 500 is accessed by selecting the parameter configuration button 232 in the left panel 202 and one of the indicator displays 502-508. The screen 500 shows all the indicator displays 502-508 that are currently active for data monitoring by scaling the indicator displays 502-508 to a smaller size suitable to fit the screen 500. For each of the indicator displays 502-508, a number of primary displayed parameters can be selected. In an embodiment, for each patient parameter, a different visual display can be used. For example, FIG. 5 uses indicator display 502 where a single parameter is selected to display. Subsequently, a real-time graphical template 514 can then be selected for configuration.

For indicator display 504, which can represent and display two parameters, a template 510 having upside-down lantern icons can be selected. Various other templates, such as a cockpit-type template 512, can also be selected. In an alternative embodiment, selection of one indicator display type applies or cascades the selection to all of the templates that are currently active. Similarly, indicator display 506 can represent and display three parameters.

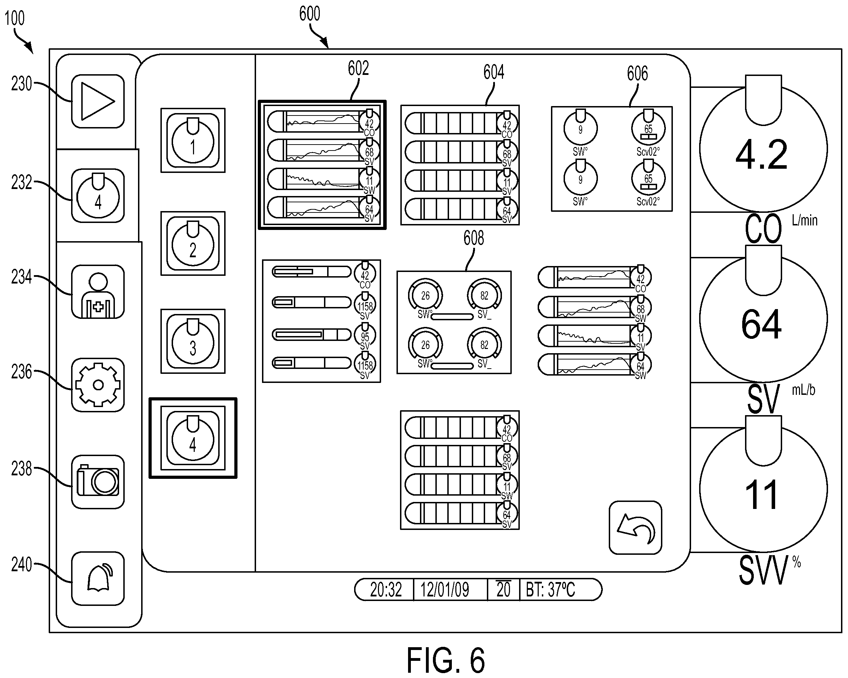

FIG. 6 is a view of a parameter configuration screen of the indicator display 508, with four primary parameters chosen for display. The parameter configuration screen 600 is accessed by selecting the parameter configuration button 232 in the left panel 202, and then the indicator display 508. The templates 602-608 allow the clinician to select a viewing style for all of the active indicator displays. The templates can be predefined and loaded into the system 101, or can be clinician-defined and stored in the memory 108 for later retrieval. For example, template 602 includes indicator displays having an upside-down lantern icon along with a real-time graphical display for each indicator display, template 604 includes indicator displays having an upside-down lantern icon along with parameter values, template 606 includes indicator displays having an upside-down lantern icon, and template 608 includes cockpit-type displays.

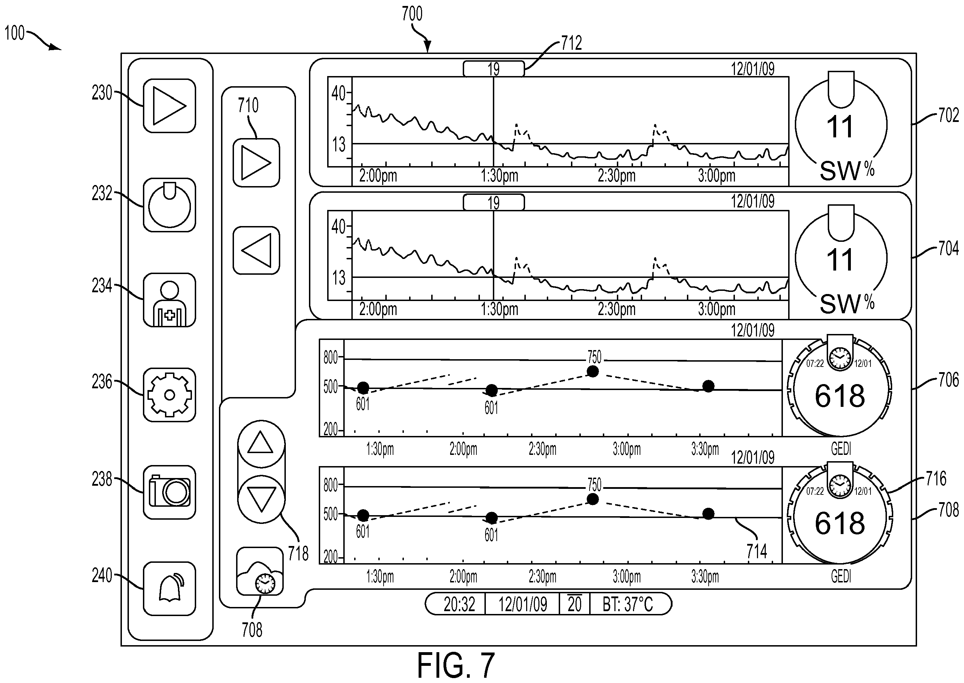

FIG. 7 is a view of a screen 700 displaying multiple patient parameters illustrating a methodology for displaying continuous information, intermittent information, and overlapping continuous/intermittent information. The system 101 allows continuous real-time data to be displayed on the screen 700, as in indicator displays 702 and 704, while also allowing the clinician to view intermittent patient data, as in indicator displays 706 and 708. In addition, continuous and intermittent data can be overlapped and displayed on the same screen 700 or on the same indicator display 702-708. In an embodiment, up to ten patient parameters can be displayed simultaneously on a screen. The placement of each indicator for each patient parameter can be selected by the clinician. For example, indicator 702 can be moved down to a position underneath indicator 704. The arrows 710 are used to move a position bar 712 to a desired position to view a patient parameter value at a specific time in the indicator displays 702 and 704. The arrows 718 are used to increase or decrease the number of intermittent parameters being displayed. For example, selecting the "up" arrow adds another intermittent parameter to the display, taking the place of a continuous parameter.

A threshold range 714 illustrates a threshold for a patient parameter value. When the patient parameter value monitored in display 708 is outside of the threshold range 714, a visual or audible alarm or indication is provided. For example, the indicator display 716 having an up-side down lantern icon can change colors to indicate that the patient parameter value is outside of the threshold range 714.

In another embodiment, indicator displays 702-708 illustrate additional information corresponding to the position of the position bar 712. For example, similar to table 248, the indicator displays 702-708 can also include a patient value reading, a percentage change between a reference point and the patient value reading, the time elapsed since the reference point, and an arrow indicating if the percentage change is negative or positive.

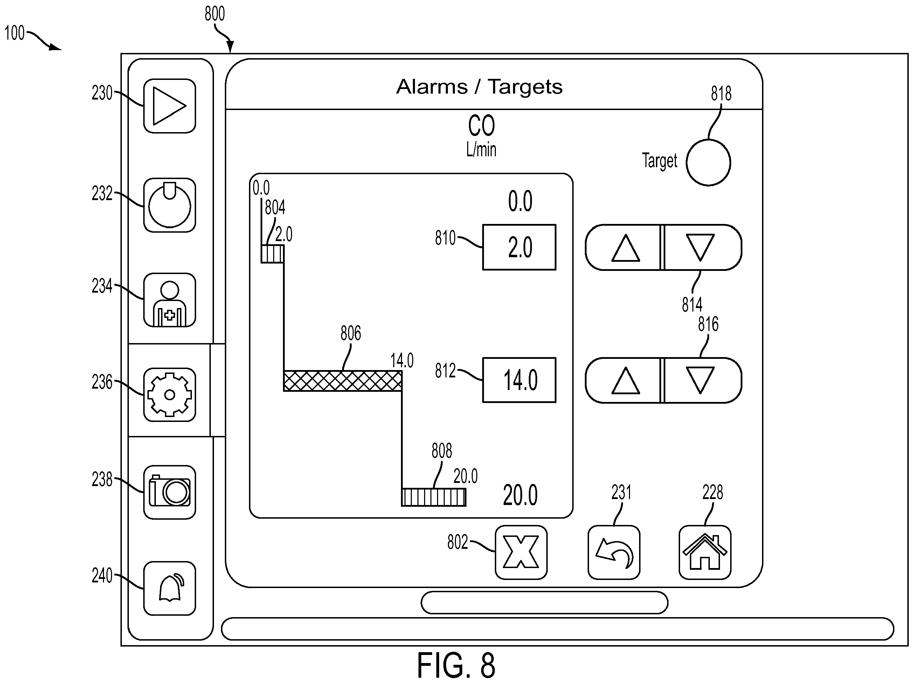

FIGS. 8-9 illustrate an alarm setting methodology. FIG. 8 is a view of an alarm/target configuration screen 800 with three threshold ranges. The alarm/target configuration screen 800 allows a clinician to set high and low thresholds for alarms and target indications. For example, the clinician may want to be notified if the CO level falls below 2.0 L/min, and if the CO level exceeds 14.0 L/min. The screen 800 includes low threshold adjustment arrows 814 and high threshold adjustment arrows 816. Selecting one of the arrows 814 adjusts a low threshold range 804 incrementally, which selecting the number buttons 810 or 812 allows input by a number pad. The low threshold range 804 can be colored red to indicate an abnormal value range. Selecting one of the arrows 816 adjusts a high threshold range 808. The high threshold range 808 can also be colored red to indicate an abnormal value range. The "target" value range 806 is between the high and low threshold ranges, and can be colored green or blue to indicate a normal patient's status. In an embodiment, the screen 800 includes a cancel button 802 which allows the clinician to exit the screen 800 without setting an alarm or target indication.

In another embodiment, a pre-determined list of alarms and target indications can be stored in the memory 108 of the system 101. For example, for the CO patient parameter, the clinician can select from a list of pre-determined alarm threshold ranges, each alarm threshold range corresponding to a specific clinical event.

In another embodiment, screen 800 displays alarm/target information for multiple parameters. For example, parameters cardiac index (CI), systolic volume index (SVI), stroke volume variation (SW), and systemic vascular resistance index (SVRI) may be displayed in screen 800. In an embodiment, the desired parameter is touched using a touch screen to zoom in and modify levels for the target, warning, and alarm settings. In another embodiment, all the parameters are modified using a configuration button. Additionally, the screen 800 can illustrate whether the alarm setting is a default setting or has been modified from the default setting. In one embodiment, the screen 800 displays a right panel 204 having real-time parameter information.

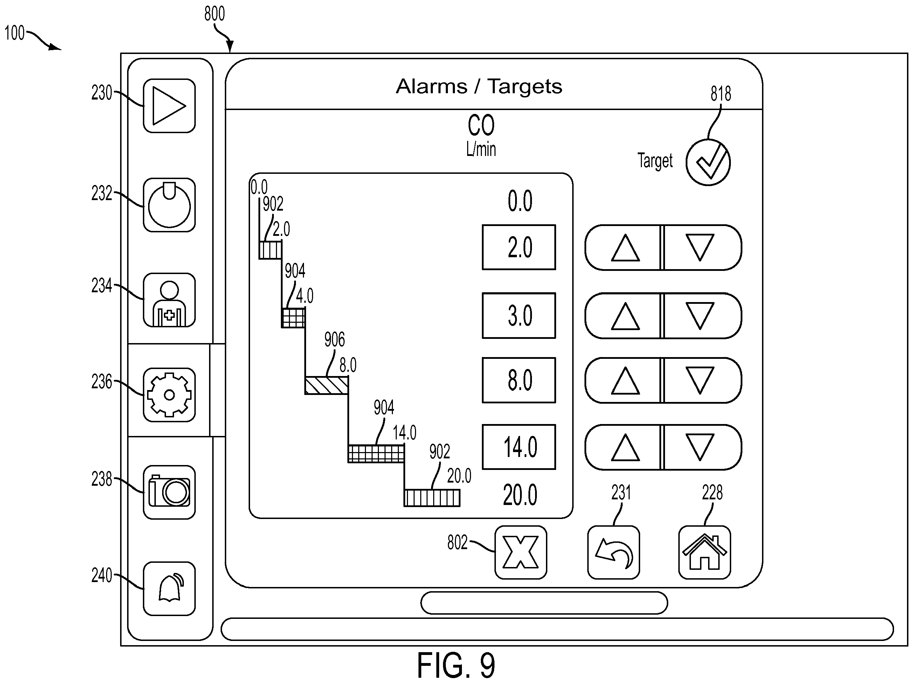

In an embodiment, the clinician can select and deselect a target option 818. Deselecting the target option 818, as illustrated in FIG. 8, creates two levels of patient's status indication: (1) outside alarm range - red, and (2) within alarm range - grey. In contrast, selecting the target option 818, as illustrated in FIG. 9, provides three levels of patient's status: (1) within target range - green, (2) outside target range and within alarm range - yellow, and (3) outside alarm range - red.

FIG. 9 is a view of an alarm/target configuration screen with five threshold ranges. In this embodiment, the clinician can set multiple threshold ranges for an alarm or target indication. For example, the clinician can set alarm ranges 902, warning ranges 904, and a target range 906. As described above, the indicator displays exhibit different behavior if the patient parameter is within the target range, outside the alarm range, or between the target range and the alarm range.

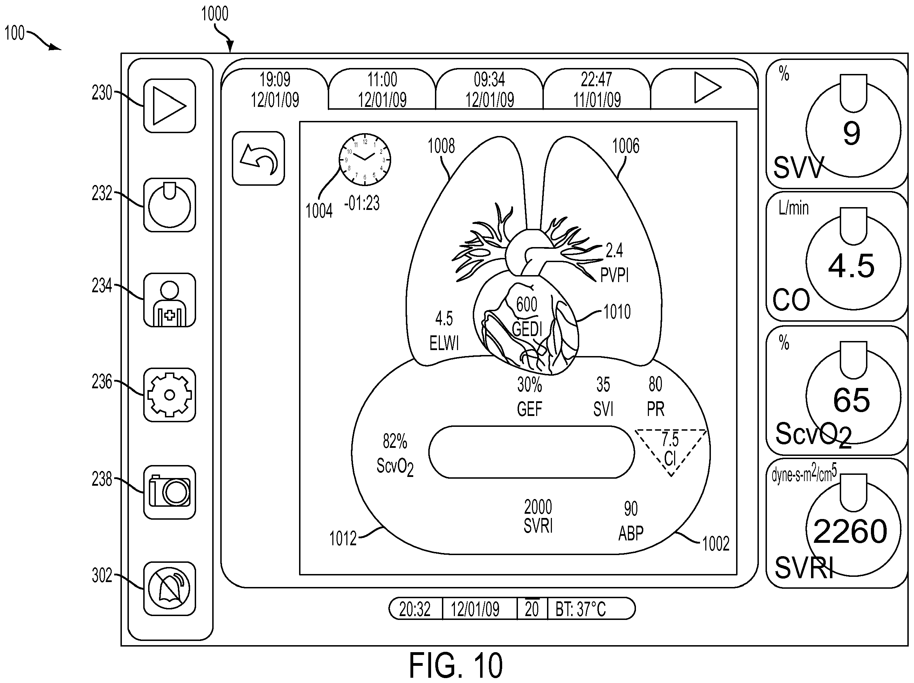

FIG. 10 is a view of a physiological indicator display screen 1000. The physiology indicator display screen 1000 displays parameter information by using physiological/anatomical shapes or by using animation. Advantageously, this feature allows the clinician to quickly and easily determine a patient's status because the clinician can easily determine what is happening to the patient through visual depictions of the patient's organ. The clinician does not need to analyze the numbers to determine what is happening to the patient, but instead can see it visually depicted on the screen as images. This can allow, for example, clinicians which may not have had as much extensive medical training to additionally bring issues to a more experienced clinician's attention. The analysis of the patient would not rest solely on the more experienced clinician, but also the experienced clinician and the clinician without the extensive medical training. Thus, the present invention, can allow for a more accurate analysis of the patient. For example, by using the anatomical shape to display parameter information, changes in parameter information are displayed graphically by changing the anatomical size or shape. The physiology indicator display screen 1000 can also use animation, other than size/shape changes, to display parameter information. For example, movement of objects can be used to simulate circulation or body functions. The objects can be, for example, bubbles to simulate blood flow.

The physiological indicator display screen 1000 includes an anatomical representation 1002 of the patient. In one embodiment, the representation 1002 includes lungs 1006 and 1008, a heart 1010, a circulatory system 1012, and/or a timer 1004. The timer 1004 can be an analog or digital clock, and can represent the time at which the parameter values were measured. The circulatory system can also be referenced, for example, as the vascular track. Various patient parameters and especially hemodynamic parameters, such as, but not limited to, extravascular lung water index (ELWI), pulmonary vascular permeability index (PVPI), global end-diastolic index (GEDI), global ejection fraction (GEF), systolic volume index (SVI), arterial blood pressure (ABP), cardiac index (CI), systemic vascular resistance index (SVRI), peripheral resistance (PR), and central venous saturation (ScvO₂) are displayed on the anatomical representation 1002. In an embodiment, the anatomical representation 1002 dynamically changes based on real-time patient parameter data, and can simulate activity of a moving heart and circulatory system. Different portions of the anatomical representation 1002 can have different colors or changing colors to indicate normal, alert, and alarm statuses.

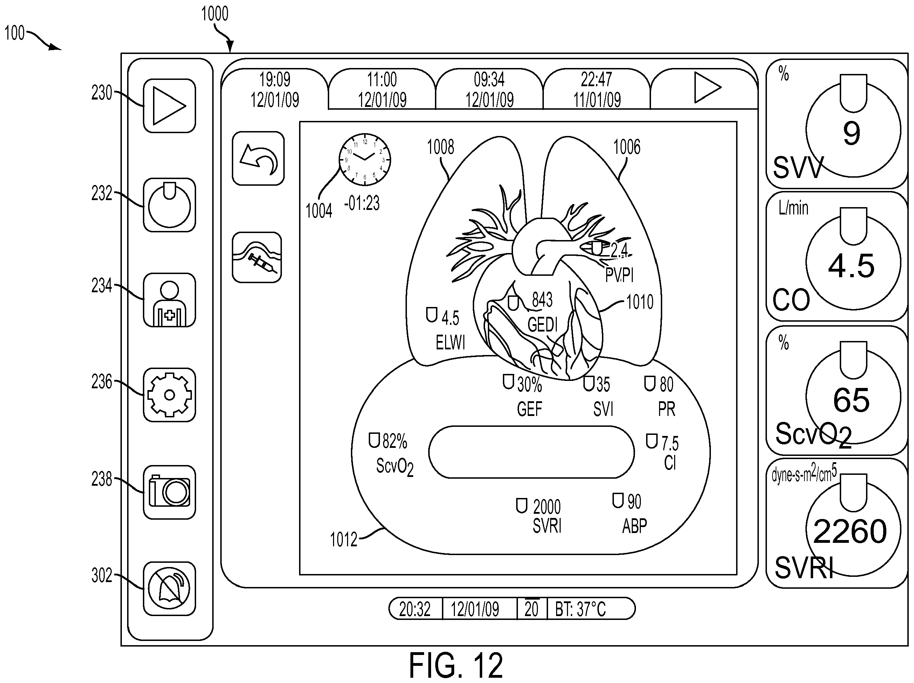

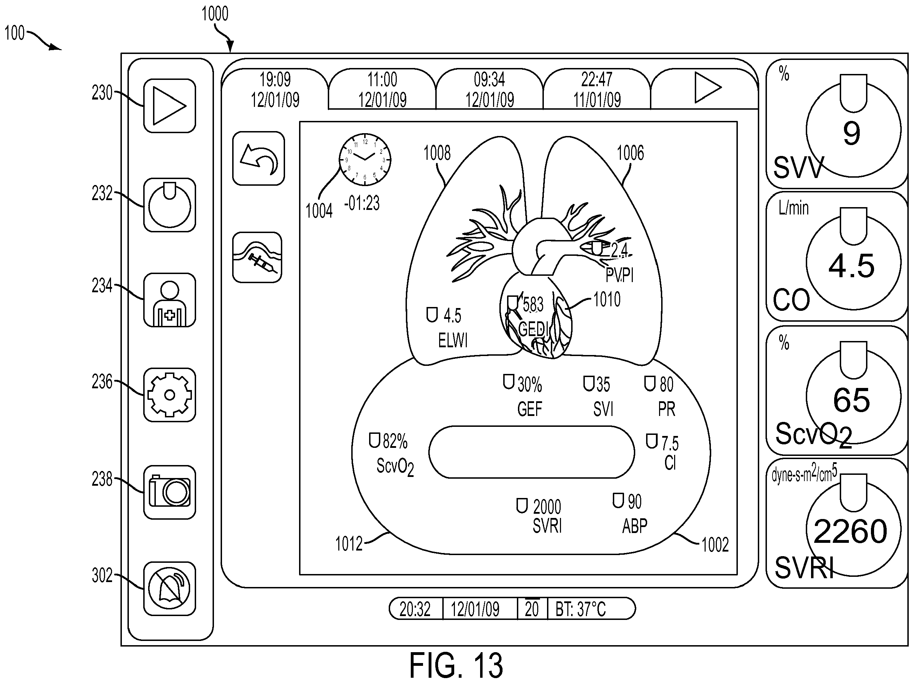

In one embodiment, the heart 1010 changes size corresponding to a change in GEDI, such that an increase in the GEDI increases the size of the graphical representation of the heart 1010 and a decrease in the GEDI decreases the size of the graphical representation of the heart 1010. This can be seen, for example, in FIGS. 12 and 13. In FIG. 10, the heart 1010 has a GEDI of 600. However, in FIG. 12, the heart 1010 has a GEDI of 843 and the size of the heart 1010 increases along with the increase in GEDI. Likewise, in FIG. 13, the heart has a GEDI of 583, and the size of the heart 1010 decreases along with the decrease in GEDI. Although the heart 1010 changes size, any other organ can also be depicted as changing its size. For example, the lungs 1008 and/or 1006 singularly or in combination can change size to reflect the condition of the patient.

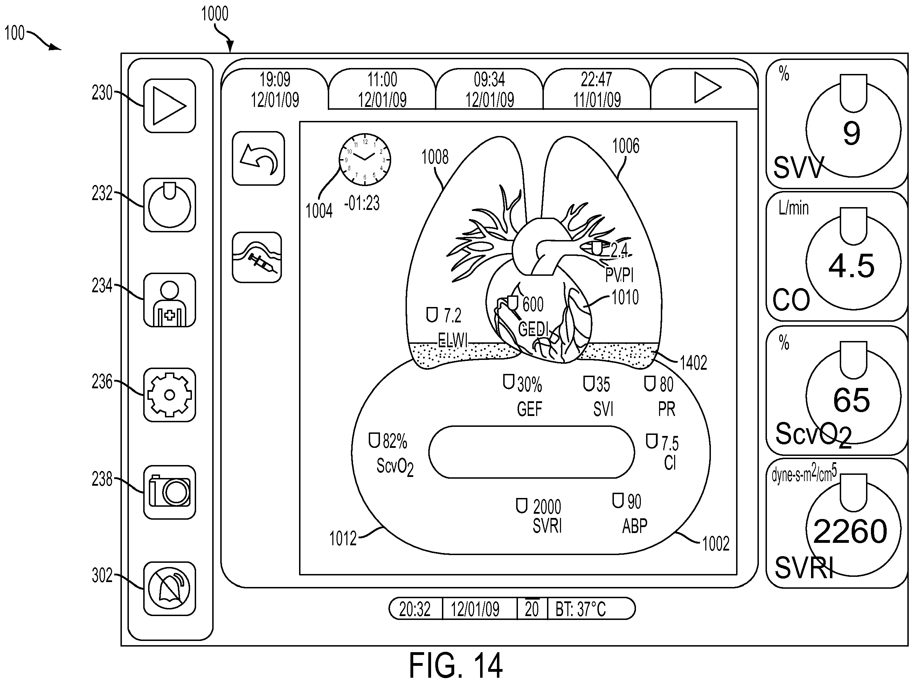

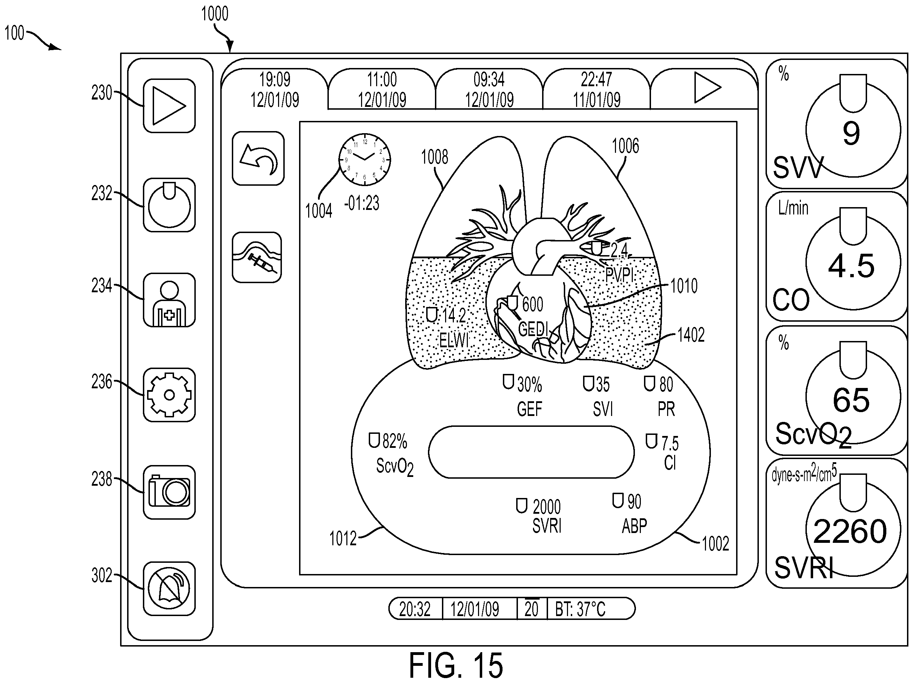

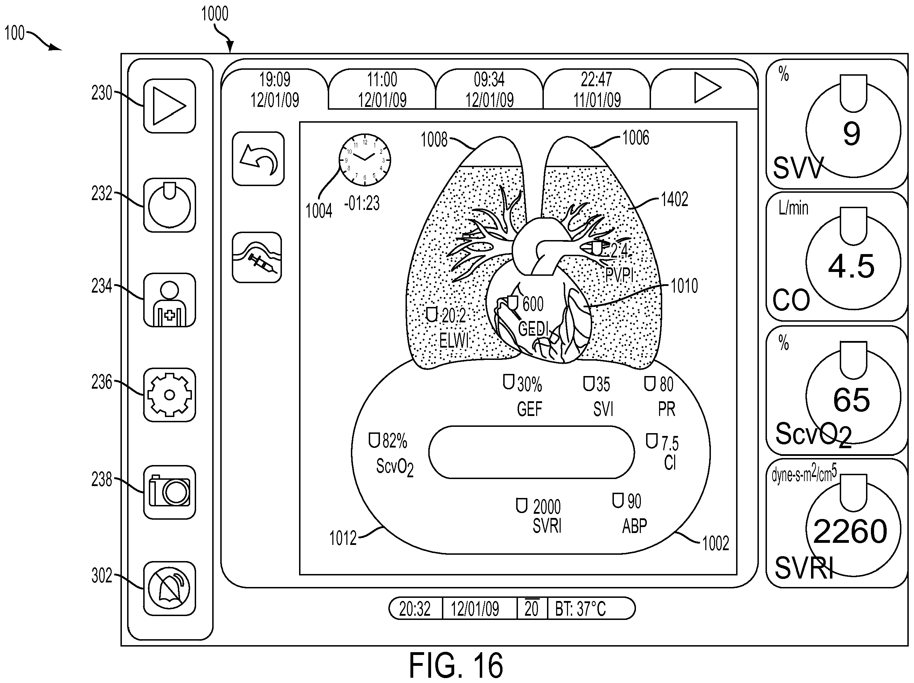

In another embodiment, the lungs 1008 and 1006 fill up with water corresponding to an increase in ELWI. FIG. 10 illustrates the ELWI having a value of 4.5 representing an amount of fluid in the lungs 1008 and 1006. In one embodiment, the ELWI value increases, representing more fluid in the lungs 1008 and 1006, and this change can be graphically displayed by additional fluid 1042 in the lungs 1008 and 1006. In another embodiment, the ELWI value decreases, representing less fluid 1042 in the lungs 1008 and 1006, and this change can be graphically displayed by less fluid in the lungs 1008 and 1006. For example, as the ELWI increases, the lungs 1008 and 1006 can fill up with water as first shown with fluid 1042 in FIG. 14, then FIG. 15, and then FIG. 16. However, when ELWI value decreases, the lungs 1008 and 1006 can decrease in water as first shown with fluid 1042 in FIG. 16, then FIG. 15, and then FIG. 14. As such, the physiology indicator display screen 1000 can also use animation, other than shape changes, to display parameter information.

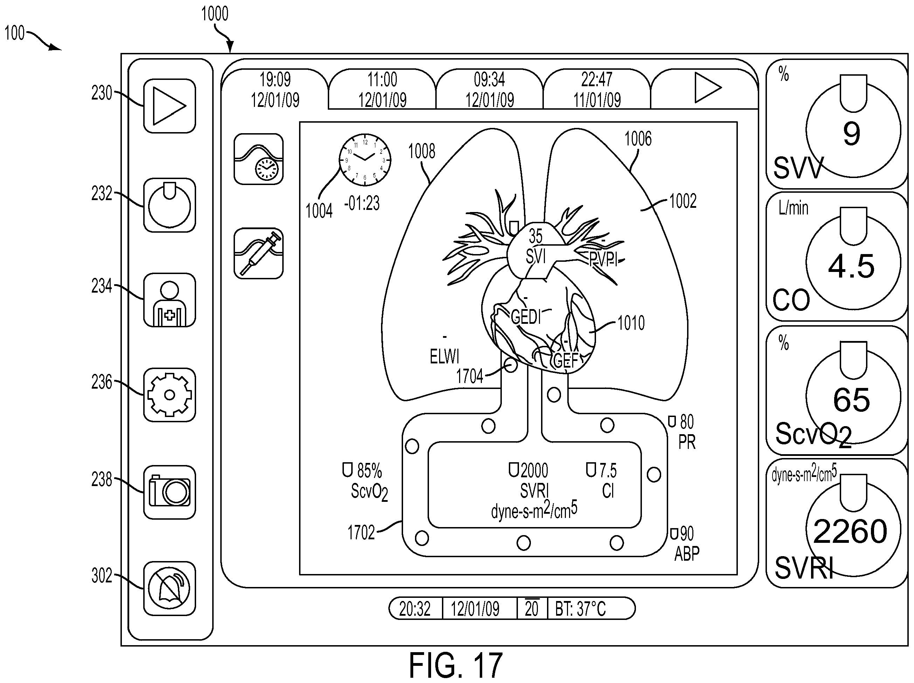

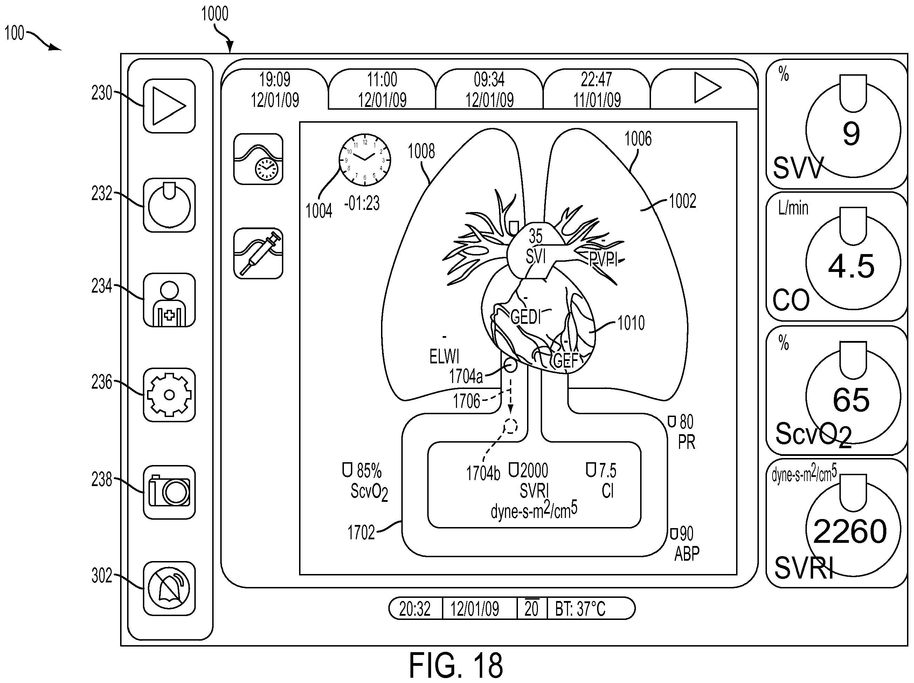

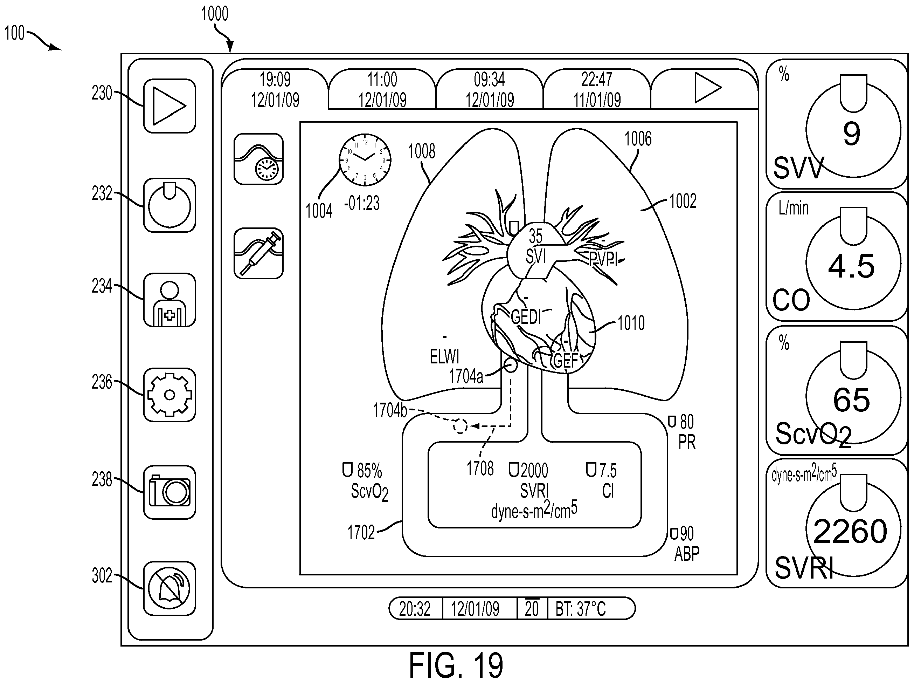

In another embodiment, the circulatory system can display animated blood cells that move at a speed corresponding to the level of cardiac output showing circulation. This can be seen, for example, in FIGS. 17, 18, and 19. In FIG. 17, the circulatory system 1702 can display animated blood cells 1704 that move at a speed corresponding to the level of cardiac output showing circulation. For example, as seen in FIG. 18, the blood cell 1704a can move to a first position at a time period indicated by the arrow 1706 and the blood cell 1704b at a first level of cardiac output. However, in FIG. 19, the blood cell 1704a can move to a second position at the same time period indicated by the arrow 1708 and the blood cell 1704b at a second level of cardiac output. In FIGS. 18 and 19, the second cardiac output is greater than the first cardiac output, thus the blood cell 1704b has traveled a longer distance in FIG. 19 when compared with FIG. 18. This can illustrate, for example, the blood cells 1704 traveling faster for the second level of the cardiac output. Although only a single blood cell 1704 is shown in FIGS. 18 and 19, the same principles can apply to all of the other blood cells 1704 which are displayed, for example, in FIG. 17.

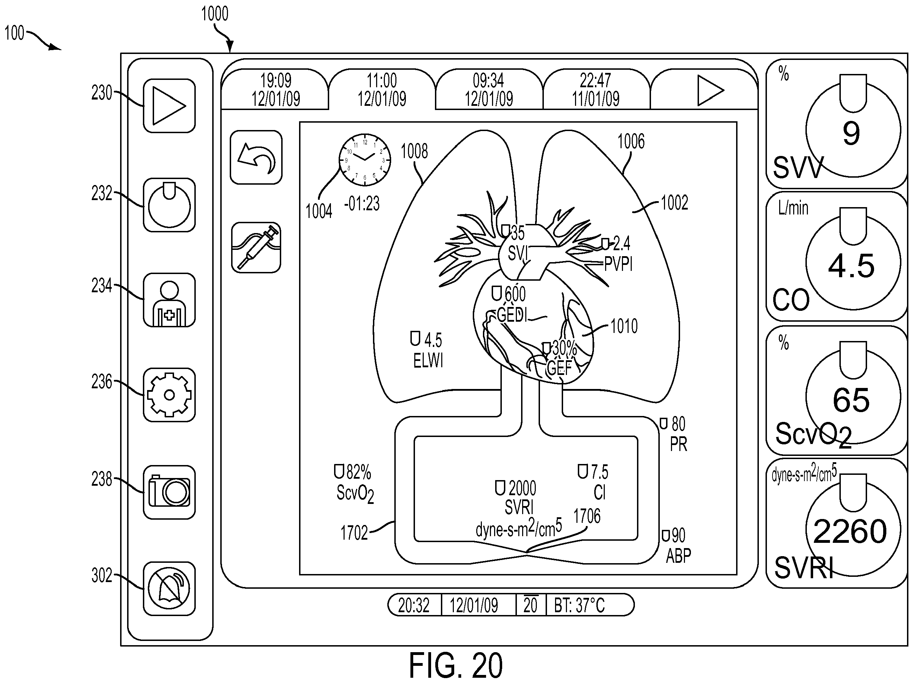

In one embodiment, the circulatory system grows and shrinks corresponding to a decrease or increase in SVRI. FIG. 10 illustrates the SVRI having a value of 2000 representing the resistance to be overcome to push blood through the circulatory system 1012. In one embodiment, the SVRI value increases, representing a higher resistance, and this increase can be graphically displayed by shrinking the width of the circulatory system as shown in FIG. 20. In FIG. 20, the circulatory system 1702 replaces the circulatory system 1012. A portion 1706 shrinks to represent the shrinking of the width of the circulatory system 1702.

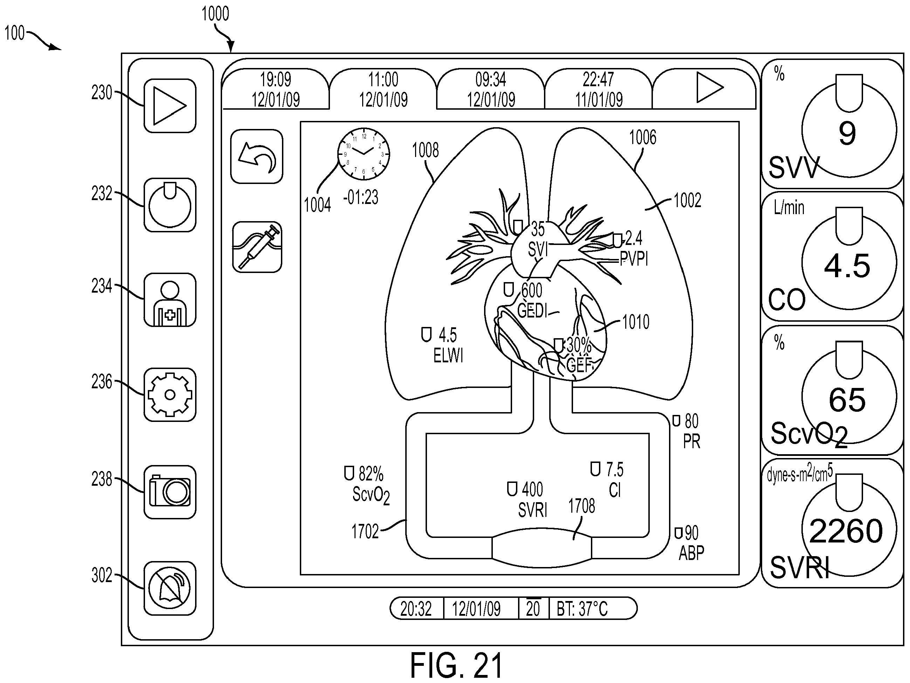

In another embodiment, the SVRI value decreases, representing a lower resistance, and this decrease can be graphically displayed by growing the width of the circulatory system 1702 as shown in FIG. 21. In FIG. 21, the circulatory system 1702 replaces the circulatory system 1012. A portion 1708 grows to represent the growing of the width of the circulatory system 1702. As such, changes to the parameter information are displayed graphically by changing the anatomical shape.

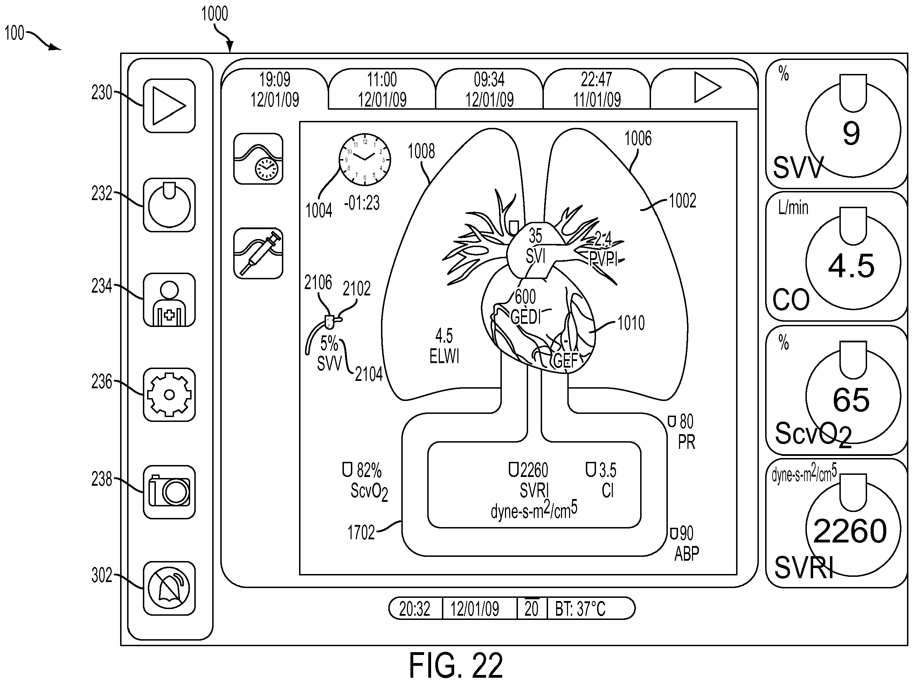

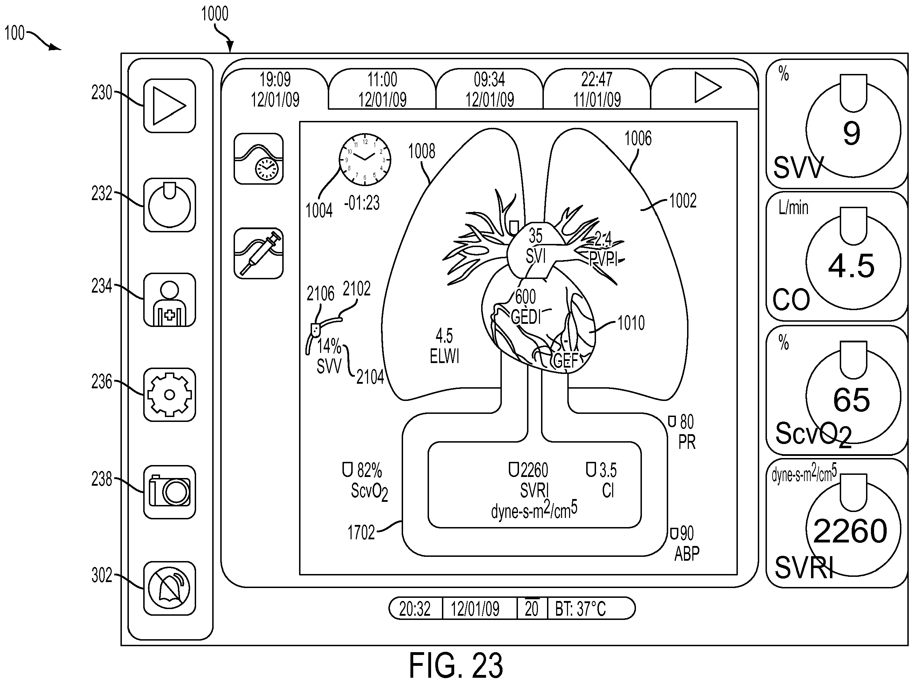

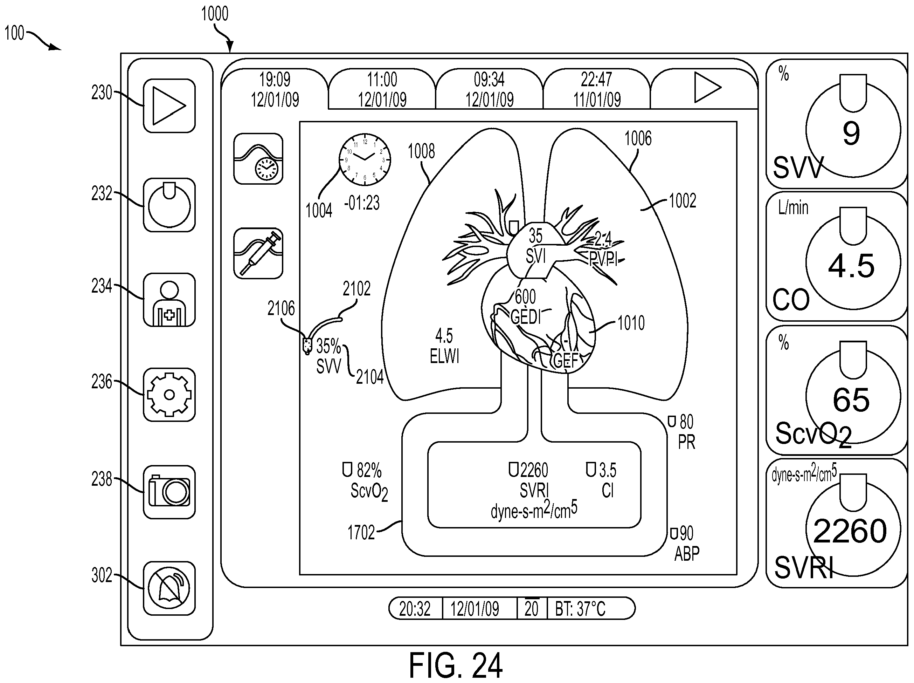

In another embodiment, the screen includes a stroke volume variation (SVV) starling curve 2102 with an indicator 2106 representing a SW value 2104 as shown in FIGS. 22 - 24. The indicator 2106 can have a first color, such as green, corresponding to the SW value 2104 being within a target range as shown in FIG. 22. The indicator 2106 can have a second color, such as yellow, corresponding to the SW value 2104 being within a warning range as shown in FIG. 23. The indicator 1016 can have a third color, such as red corresponding to the SVV value 2104 being within an alarm range as shown in FIG. 24. Furthermore, the indicator 2106 can move along the curve 1014 corresponding to the SW value 2104 as shown in FIGS. 22 - 24.

In another embodiment, a physiological relationship screen 2500 is used to display a physiological relationship between the parameters. In one embodiment, various blocks 2502 are connected together using, for example, branches illustrated by various lines 2504, 2506, and 2508. Line 2504 can be a first type of line, line 2506 can be a second type of line, and line 2508 can be a third type of line. Each type of lines can denote different relationships between the various blocks 2502. For example, the line 2504 can denote a first type of relationship between a block for ScvO₂ and the block for VO₂e. The line 2506 can denote a second type of relationship between a block for CI and the block for Pr. The line 2508 can denote a third type of relationship between a first block for SpO₂ and a second block for SpO₂.

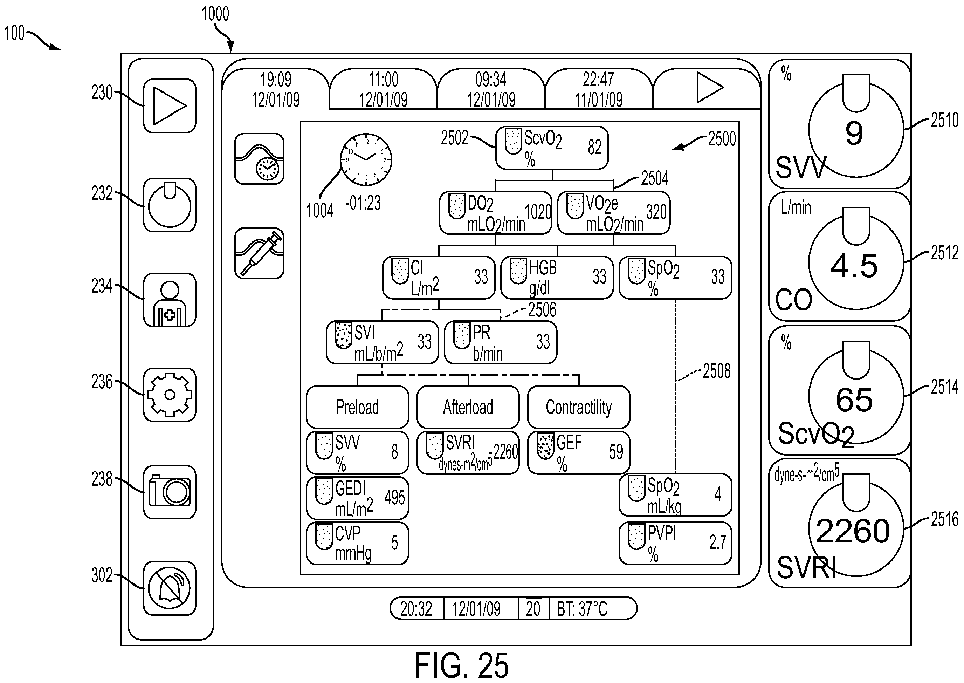

In FIG. 25, the block 2502 for ScvO₂ is on top, and branches down to the blocks 2502 for DO₂ and VO₂e. The blocks 2502 for DO₂ branches down to the blocks 2502 for the blocks CI, HGB, and SpO₂. The blocks 2502 for CI branches down to SVI and PR. In one embodiment, indicator displays 2510, 2512, 2514, and 2516, similar to the indicator displays 242, 244, and 246 in FIG. 2, have a color, such as green, yellow, and/or red. In one embodiment, the lines 2504, 2506, and 2508 also have one or more colors, the colors corresponding to the indicator display color. For example, the lines immediately above and below a parameter can display red when a corresponding indicator display is red.

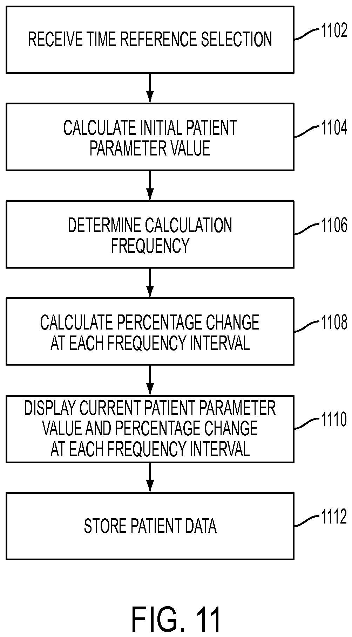

FIG. 11 is a flow diagram of the event marking and analysis method. In step 1102, the system 101 receives a time reference selection. The time reference can be made upon a clinician selecting a point in time on a time-lapse graph as described above. In another embodiment, the reference point can be scheduled ahead of time, and the system 101 automatically loads the time reference at the scheduled time without clinician intervention. In yet another embodiment, the system 101 can be coupled to a network, such as a wireless network, which allows a remote clinician to select a reference point via their computer or mobile device.

After the system 101 receives a time reference in step 1102, the initial patient parameter value(s) are calculated in step 1104. For example, the CO at the reference point time is determined. In an embodiment, the calculations can be based on pre-stored algorithms or formulas, or alternatively, the formulas can be entered by the clinician.

In step 1106, the system 101 determines the calculation frequency. For example, the clinician can select a time interval at which the system 101 calculates a parameter. Referring to FIG. 2, the time intervals are 15 minutes. In an embodiment, if the time interval is not selected, the system 101 automatically has a default frequency at which it conducts calculations and displays the calculated values.

Next, in step 1108, the percentage change at each frequency interval is calculated. After a current value is determined in step 1106, a percentage change from the initial value is determined. In an embodiment, the following formula is used to determine the percentage change: ([current value - initial value] / [initial value]) x 100.

In step 1110, the current patient parameter value and percentage change at each frequency interval is displayed, as shown in FIG. 2. In step 1112, the measured and calculated patient data is stored to the memory 108 for later retrieval.

The present disclosure is not limited to monitoring hemodynamic parameters, and can be used with any other types of patient monitoring, such as glucose monitoring, as well as other types of respiratory and cardiovascular monitoring. In such cases, the affected body parts can be displayed along with their respective images or animations. For example, for glucose monitoring, a pancreas can be displayed along with objects which depict insulin.

While the principles of the disclosure have been illustrated in relation to the exemplary embodiments shown herein, the principles of the disclosure are not limited thereto and include any modification, variation or permutation thereof.

Those skilled in the art will appreciate that the various illustrative logical blocks, modules, circuits, and algorithms described in connection with the embodiments disclosed herein may be implemented as electronic hardware, computer software, or combinations of both. To illustrate this interchangeability of hardware and software, various illustrative components, blocks, modules, circuits, and algorithms have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present disclosure.

The various illustrative logical blocks, modules, and circuits described in connection with the embodiments disclosed herein may be implemented or performed with a general purpose processing device, a digital signal processing device (DSP), an application specific integrated circuit (ASIC), a field programmable gate array (FPGA) or other programmable logic device, discrete gate or transistor logic, discrete hardware components, or any combination thereof designed to perform the functions described herein. A general purpose processing device may be a microprocessing device, but in the alternative, the processing device may be any conventional processing device, processing device, microprocessing device, or state machine. A processing device may also be implemented as a combination of computing devices, e.g., a combination of a DSP and a microprocessing device, a plurality of microprocessing devices, one or more microprocessing devices in conjunction with a DSP core or any other such configuration.

The apparatus, methods or algorithms described in connection with the embodiments disclosed herein may be embodied directly in hardware, software, or combination thereof. In software the methods or algorithms may be embodied in one or more instructions that may be executed by a processing device. The instructions may reside in RAM memory, flash memory, ROM memory, EPROM memory, EEPROM memory, registers, hard disk, a removable disk, a CD-ROM, computer-readable medium which can cause a processor to execute certain steps, or any other form of storage medium known in the art. An exemplary storage medium is coupled to the processing device such the processing device can read information from, and write information to, the storage medium. In the alternative, the storage medium may be integral to the processing device. The processing device and the storage medium may reside in an ASIC. The ASIC may reside in a user terminal. In the alternative, the processing device and the storage medium may reside as discrete components in a user terminal.

The previous description of the disclosed embodiments is provided to enable any person skilled in the art to make or use the present disclosure. Various modifications to these embodiments will be readily apparent to those skilled in the art, and the generic principles defined herein may be applied to other embodiments without departing from the scope of the disclosure. Thus, the present disclosure is not intended to be limited to the embodiments shown herein but is to be accorded the widest scope consistent with the principles and novel features disclosed herein.
The invention may be embodied in other specific forms without departing from its essential characteristics. The described embodiments are to be considered in all respects only as illustrative and not restrictive and the scope of the invention is, therefore, indicated by the appended claims rather than by the foregoing description.

## Claims

1. A system (101) for providing a physiological representation of a patient, comprising:
a sensor (110) configured to monitor a physiological parameter of a patient corresponding to an organ of the patient and provide an output signal corresponding to the monitored physiological parameter;
a processing unit (106) and a display device (100); wherein
the organ comprises the heart of the patient; and
the processing unit is configured to cause the display device to display the organ, and to display a shape change of the organ or an animation of the organ based on the output signal;
**characterized in that**
the processing unit is further configured to cause the display device (100) to display a stroke volume variation starling curve (2102) with an indicator (2106) representing a stroke volume variation value (2104), the indicator moving along the stroke volume variation starling curve corresponding to the stroke volume variation value.

2. The system (101) of claim 1, wherein the shape change comprises the heart changing sizes based on the output signal.

3. The system (101) of claim 1, wherein the display device (100) is further configured to display a circulatory system, and further configured to display, adjacent to the circulatory system, an output signal corresponding to the circulatory system.

4. A computer-readable medium storing a program for providing a physiological representation of a patient, which when executed by a system comprising a sensor, a processing unit and a display device causes the processing unit to:
monitor a physiological parameter of a patient corresponding to a heart of the patient;
provide an output signal corresponding to the monitored physiological parameter; and
display the heart and a shape change of the heart, or an animation of the heart based on the output signal;
**characterized in that**
the processing unit is further caused to display a stroke volume variation starling curve (2102) with an indicator (2106) representing a stroke volume variation value (2104), the indicator moving along the stroke volume variation starling curve corresponding to the stroke volume variation value.

## Patentansprüche

1. System (101) zum Bereitstellen einer physiologischen Darstellung eines Patienten, umfassend:
einen Sensor (110), der ausgestaltet ist, um einen physiologischen Parameter eines Patienten zu überwachen, der einem Organ des Patienten entspricht, und um ein Ausgabesignal bereitzustellen, welches dem überwachten physiologischen Parameter entspricht;
eine Verarbeitungseinheit (106) und eine Anzeigevorrichtung (100); wobei
das Organ das Herz des Patienten umfasst; und
die Verarbeitungseinheit ausgestaltet ist, um zu bewirken, dass die Anzeigevorrichtung basierend auf dem Ausgabesignal das Organ anzeigt und eine Formveränderung des Organs anzeigt oder eine Animation des Organs basierend auf dem Ausgabesignal anzeigt;
**dadurch gekennzeichnet, dass**
die Verarbeitungseinheit des Weiteren ausgestaltet ist, um zu bewirken, dass die Anzeigevorrichtung (100) eine Starling-Kurve der Herzschlagvolumenvariation (2102) mit einem Indikator (2106) anzeigt, der einen Wert der Herzschlagvolumenvariation (2104) darstellt, wobei der Indikator sich entlang der Starling-Kurve der Herzschlagvolumenvariation entsprechend dem Wert der Herzschlagvolumenvariation bewegt.

2. System (101) nach Anspruch 1, wobei die Formänderung die sich ändernden Größen des Herzens basierend auf dem Ausgabesignal umfasst.

3. System (101) nach Anspruch 1, wobei die Anzeigevorrichtung (100) des Weiteren ausgestaltet ist, um ein Kreislaufsystem anzuzeigen, und des Weiteren ausgestaltet ist, um benachbart zu dem Kreislaufsystem ein Ausgabesignal anzuzeigen, welches dem Kreislaufsystem entspricht.

4. Computerlesbares Medium, welches ein Programm zum Bereitstellen einer physiologischen Darstellung eines Patienten speichert, das bei Ausführung durch ein System, welches einen Sensor, eine Verarbeitungseinheit und eine Anzeigevorrichtung umfasst, bewirkt, dass die Verarbeitungseinheit folgendes ausführt:
Überwachen eines physiologischen Parameters eines Patienten, der einem Herzen des Patienten entspricht;
Bereitstellen eines Ausgabesignals, welches dem überwachten physiologischen Parameter entspricht; und
Anzeigen des Herzens und einer Formveränderung des Herzens oder einer Animation des Herzens basierend auf dem Ausgabesignal;
**dadurch gekennzeichnet, dass**
des Weiteren bewirkt wird, dass die Verarbeitungseinheit eine Starling-Kurve der Herzschlagvolumenvariation (2102) mit einem Indikator (2106) anzeigt, der einen Wert der Herzschlagvolumenvariation (2104) darstellt, wobei der Indikator sich entlang der Starling-Kurve der Herzschlagvolumenvariation entsprechend dem Wert der Herzschlagvolumenvariation bewegt.

## Revendications

1. Système (101) pour obtenir une représentation physiologique d'un patient, comprenant :
un capteur (110) conçu pour surveiller un paramètre physiologique d'un patient correspondant à un organe du patient et pour fournir un signal de sortie correspondant au paramètre physiologique surveillé ;
une unité de traitement (106) et un dispositif d'affichage (100) ;
l'organe comprenant le cœur du patient ; et
l'unité de traitement étant conçue pour amener le dispositif d'affichage à afficher l'organe et à afficher un changement de forme de l'organe ou une animation de l'organe sur la base du signal de sortie ;
**caractérisé en ce que**
l'unité de traitement est en outre conçue pour amener le dispositif d'affichage (100) à afficher une courbe de Starling de variation du volume systolique (2102) avec un indicateur (2106) représentant une valeur de la variation du volume systolique (2104), l'indicateur se déplaçant le long de la courbe de Starling de variation du volume systolique correspondant à la valeur de la variation du volume systolique.

2. Système (101) selon la revendication 1, le changement de forme comprenant les dimensions de changement du cœur sur la base du signal de sortie.

3. Système (101) selon la revendication 1, le dispositif d'affichage (100) étant en outre conçu pour afficher un système circulatoire et en outre conçu pour afficher, à côté du système circulatoire, un signal de sortie correspondant au système circulatoire.

4. Support lisible par ordinateur stockant un programme pour obtenir une représentation physiologique d'un patient qui, lorsqu'il est exécuté par un système comprenant un capteur, une unité de traitement et un dispositif d'affichage, amène l'unité de traitement à :
surveiller un paramètre physiologique d'un patient correspondant au cœur du patient ;
fournir un signal de sortie correspondant au paramètre physiologique surveillé ; et
afficher le cœur et un changement de forme du cœur ou une animation du cœur sur la base du signal de sortie ;
**caractérisé en ce que**
l'unité de traitement est en outre amenée à afficher une courbe de Starling de variation du volume systolique (2102) avec un indicateur (2106) représentant une valeur de la variation du volume systolique (2104), l'indicateur se déplaçant le long de la courbe de Starling de variation du volume systolique correspondant à la valeur de la variation du volume systolique.
